# EUROPEAN PATENT SPECIFICATION

(11) **EP 2 341 972 B1**
(45) Date of publication and mention of the grant of the patent: **01.04.2015**
(21) Application number: 09744763.5
(22) Date of filing: 19.10.2009
(51) Int. Cl.: A61M 25/00, A61M 27/00

(54) **CATHETER STRUCTURE**
KATHETERKONSTRUKTION
STRUCTURE DE CATHÉTER

(30) Priority: 20.10.2008 IT MI20080348 U
(43) Date of publication of application: 13.07.2011
(73) Proprietor: Rocco, Francesco, 20135 Milano (IT)
(72) Inventor: Rocco, Francesco, 20135 Milano (IT)
(74) Representative: Lampis, Marco
(86) International application number: PCT/IB2009/054580
(87) International publication number: WO 2010/046828

(56) References cited:
- WO-A-2007/005734
- WO-A-2008/081497
- US-A1- 2008 071 250
- US-B1- 6 837 868

## Description

The present invention relates to a catheter structure equipped with an adjustable self-locating device.

In the medical sector and in particular in urology catheters are frequently used to help drain substances, including urine.

Said catheters are composed essentially of a flexible tube which must be introduced into the urethra as far as the bladder or into another cavity and which has, at the end introduced into the cavity, one or more drainage holes communicating with the central channel so as to convey the fluids outside the organ.

After introduction, the catheter must be fixed inside the cavity to be drained (bladder or kidneys) so that drainage of the liquids is performed efficiently and the catheter does not move from the correct position.

Fixing is achieved by means of an adjustable self-locating device.

Usually the adjustable self-locating device is in the form of a small balloon positioned at one end of the flexible tube.

During insertion and removal the balloon is deflated so as to facilitate manoeuvring. The balloon is inflated, by supplying a suitable inflation fluid, when the end of the flexible tube is situated inside the cavity to be drained.

Fixing is obtained by resting the bottom surface of the balloon on the adjacent inner surface of the cavity to be drained.

The balloon consists of a membrane which is expandable substantially in the radial direction and can be connected to an inflation fluid supply source via a duct formed in the structure of the flexible tube.

Drainage of the urine is made possible owing to the presence of a hole situated in the vicinity of the end of the flexible tube, in particular situated between the end of the flexible tube and the balloon.

As a result of this location of the drainage hole, a zone is created inside the cavity to be drained, situated between the hole and the bottom surface of the balloon (or the inner surface of the cavity to be drained), where the urine cannot be discharged and therefore stagnates.

WO 2008/081497 discloses a catheter in which two holes are above the bottom surface of the balloon.

Stagnation of urine, as is known, may create various problems in the patient, for example infections of a bacterial nature.

In the case of prolonged catheterism the stagnating urine may result in the formation of limescale on the outer surface of the flexible tube, which limescale, during extraction of the catheter, may cause damage to or small tears in the wall of the urinary duct.

These drawbacks have been partially overcome by a further type of catheter (industrial utility model No. 21426 B/77) which envisages the use of two separate diametral chambers in place of the balloon.

The two diametral chambers are positioned in a manner similar to that already seen for the balloon, in the vicinity of the end of the flexible tube.

Each chamber consists essentially of a membrane which can be expanded outwards by means of the action of a suitable inflation fluid.

In particular one of the two chambers is supplied directly via a duct entirely similar to that of the preceding solution.

The other chamber is supplied via a small channel which connects it to the preceding chamber. Usually the channel has a direction perpendicular to the axis of the flexible tube and is associated with the external structure of the flexible tube.

In this solution, drainage of the liquid is performed via two holes, which usually have a long shape in the direction of the axis of the flexible tube.

The two holes are situated in opposite positions and on the surface of the flexible tube not covered by the two diametral chambers.

This solution has a first drawback associated with its structure and in particular with the industrial production thereof.

From a constructional point of view, the assembly composed of the two diametral chambers and the channel is not easy to manufacture since the diametral dimensions of the catheter are very small.

From an operational point of view, supplying of the inflation fluid to a single diametral chamber, with supplying of the second chamber being performed by the small channel, may result in a pressure imbalance between the two chambers.

In particular, in the chamber which is supplied directly, a pressure greater than that of the second chamber is created owing to local losses at the inlet and outlet and general losses due to the extremely small diameter of the channel.

The pressure imbalance results in varying volumes of the two diametral chambers and therefore incorrect positioning of the end of the flexible tube inside the cavity to be drained.

Alternatively two ducts have been used for supplying the inflation fluid, i.e. one for each chamber, with obvious constructional complications and difficulty in obtaining an equal pressure.

WO 2007/005734 discloses catheters in which one U-shaped balloon is inflated around a U-shaped tip of the catheter or two separated balloons are inflated from two lateral arms of the U-shaped tip of the catheter.

US 6.837.868 discloses a catheter having one or more inflatable flat rectangular envelopes each of them connected on the side of the catheter wall along a single line which is parallel the catheter axis.

US 2008/0071250 discloses catheters which have one symmetrical balloon around the tube or a plurality of balloons.

The object of the present invention is therefore to overcome the drawbacks of the prior art and in particular to provide a catheter structure which allows correct and complete drainage of the liquid from the cavity to be drained.

A further object of the present invention is to obtain a catheter structure comprising an adjustable self-locating device which may be easily produced on an industrial scale and which does not have the defects of the prior art.

These and other objects are achieved by a catheter structure according to Claim 1. The further advantages and characteristic features of the present invention will become clear from the detailed description which follows of a number of examples of embodiment, provided by way of a non-limiting example, with reference to the accompanying drawings in which:
Fig. 1 shows a longitudinally sectioned view of a catheter structure according to the present invention;
Fig. 2 shows a longitudinally sectioned view of a catheter structure according to the present invention, rotated through 90° compared to Figure 1;

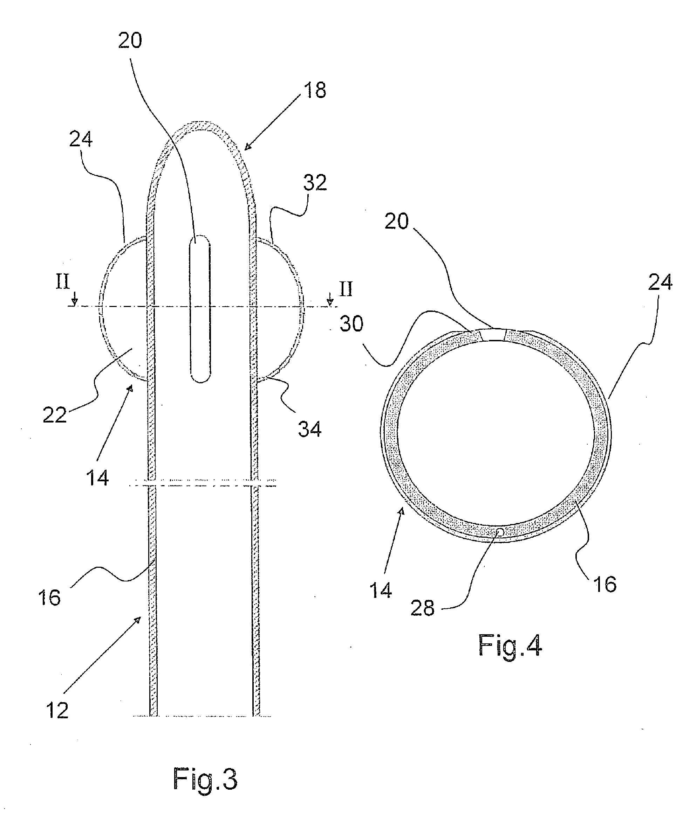

Fig. 3 shows a longitudinally sectioned view of a catheter structure according to the present invention with the diametral chamber expanded;
Fig. 4 shows a cross-sectional view, along the plane I-I of Figure 2, of a catheter structure according to the present invention;
Fig. 5 shows a cross-sectional view, along the plane II-II of Figure 3, of a catheter according to the present invention with the diametral chamber expanded;
Fig. 6 shows a perspective view of a catheter according to the present invention;
Fig. 7 shows a perspective view of a catheter according to the present invention with the diametral chamber expanded;
Fig. 8 shows a perspective view of a catheter according to the present invention, rotated through 180° compared to Figure 7, with the diametral chamber expanded;
Figure 9 shows a partial perspective view of an alternative embodiment of a catheter according to the invention;
Fig. 10 shows a cross-sectional view of the catheter according to Figure 9;
Figure 11 shows a partial perspective view of a further alternative embodiment of a catheter according to the invention;
Fig. 12 shows a cross-sectional view of the catheter according to Figure 11.

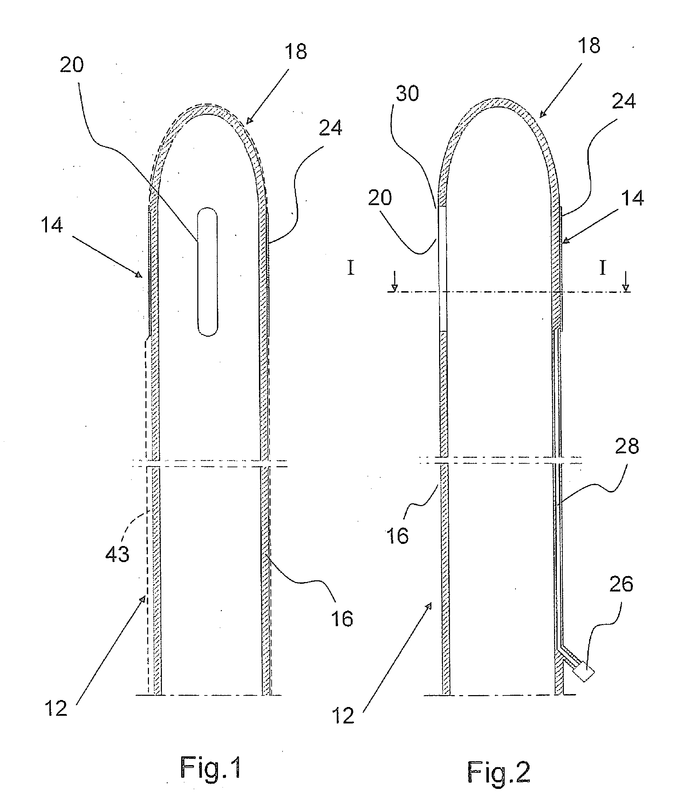

Figures 1, 2 and 5 show a catheter structure 12 equipped with an adjustable self-locating device 14 and comprising a flexible tube 16 which has one end 18 intended to be introduced into the cavity to be evacuated.

With reference to the flexible tube 16 the following are specifically defined:
- an axial direction identified by any straight line parallel to the axis of the flexible tube 16;
- a radial direction perpendicular to the axial direction and passing through the axis of the flexible tube 16.

From hereon, reference will be made to a transverse plane as the plane perpendicular to the axial direction.

The end 18 of the tube 16 comprises a hole 20 and a diametral chamber 22 formed by a membrane 24 expandable substantially outwards.

The diametral chamber 22 surrounds over a predetermined height the outer surface of the tube 16.

Moreover, the diametral chamber 22 is connected to the means for supplying and controlled discharging of the pressurised fluid (indicated schematically in Figure 2 by the number 26) via a duct 28 formed in the structure of the tube 16.

The diametral chamber 22 consists of a circular rim portion concentric with the flexible tube 16 and defined by the membrane 24, establishing a surface zone 30 of the flexible tube 16 without diametral chamber 22 in which the hole 20 is formed.

The diametral chamber 22 in the expanded condition defines specifically an upper surface 32 directed towards the end 18 of the tube 16 and a lower surface 34 opposite to the first surface.

The lower surface 34 is the surface of the diametral chamber 22 which, in the operating condition, engages with the wall of the cavity to be drained, resulting in the self-locating action.

The hole 20 has the form of an ellipsoid elongated in the axial direction and in particular extending between the limit of the lower surface and upper surface of the diametral chamber 22.

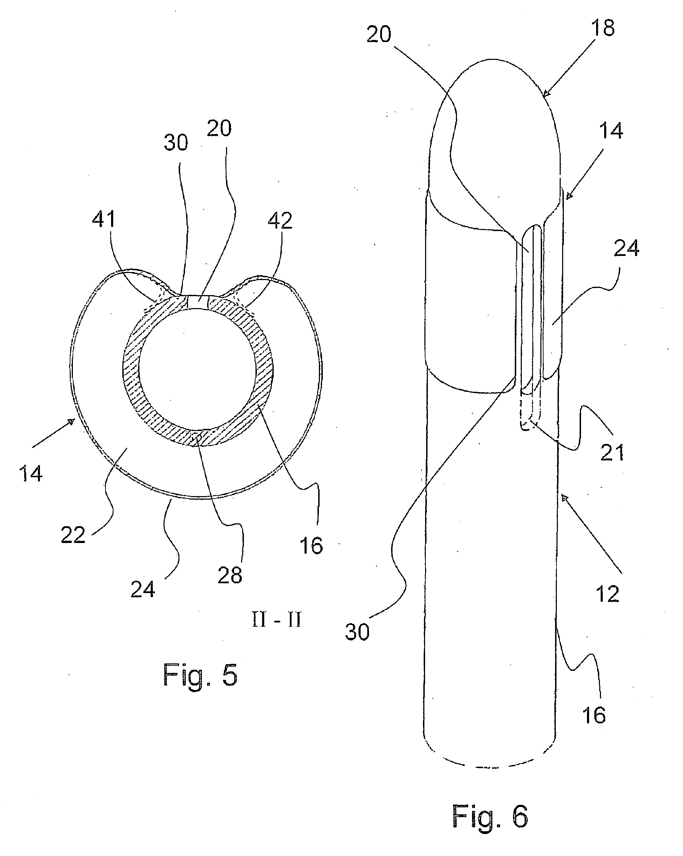

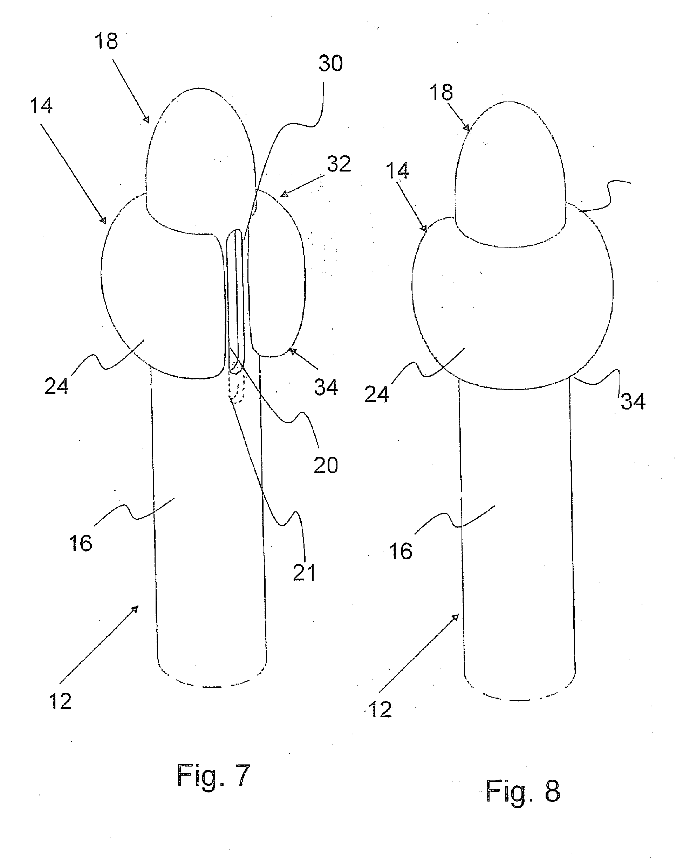

In a further embodiment, indicated by broken lines in Figures 6 and 7, the hole 20 may extend from the limit of the upper surface 32 of the chamber 22 to a point beyond the limit of the lower surface 34 of the chamber 22.

The reference number 21 indicates the hole portion 20 situated underneath the level of the surface 34.

In this way, in the operating condition, the hole 20 extends both inside the cavity to be drained and also along the urethral canal.

In one possible embodiment the diametral chamber 22 is formed by a single membrane 24 which is fixed (welded or glued) in a manner known per se to the outer surface of the tube 16.

The membrane 24 is therefore welded along its edges which extend in the transverse plane, along two circumferences which are spaced in the axial direction. It is also welded along a surface portion having an axial length equal to or greater than the axial length of the chamber 22 and transverse width to be chosen depending on the width of the hole 20, therefore defining the zone 30 not occupied by the chamber 22.

In a second embodiment the membrane 24 consists of a flat element which is wound partially around the tube 16, the end edges thereof being welded or glued in the same manner as in the solution described above, in positions spaced by a given arc in the transverse plane.

Therefore the zone 30 not occupied by the chamber 22 is defined by two axial sides, which are parallel to the axial direction, and by two arcs of a circumference belonging to two transverse planes.

With reference to Figures 5 and 6, it can be seen how each end edge of the membrane 24 is fixed to the underlying surface of the flexible tube not along a line, but along a superficial arc in the transverse plane, so as to ensure greater resistance to separation of the membrane from the wall of the underlying flexible tube, when the membrane 24 is expanded to form the chamber 22.

One consequence of this type of weld is that, with respect to a transverse plane, the angle formed by a straight line tangential to the membrane and a straight line tangential to the tube 16, in the zone where the membrane 24 is separated from the tube, will always be less than 90°.

As shown in broken lines in Figure 5, the end edges 41, 42 for fixing the membrane along the slit 20 may also be directed towards the inside of the chamber 22.

The self-locating device may also be formed with the inflation chamber separate from the catheter walls, so as to facilitate for example sealing of the inflation chamber.

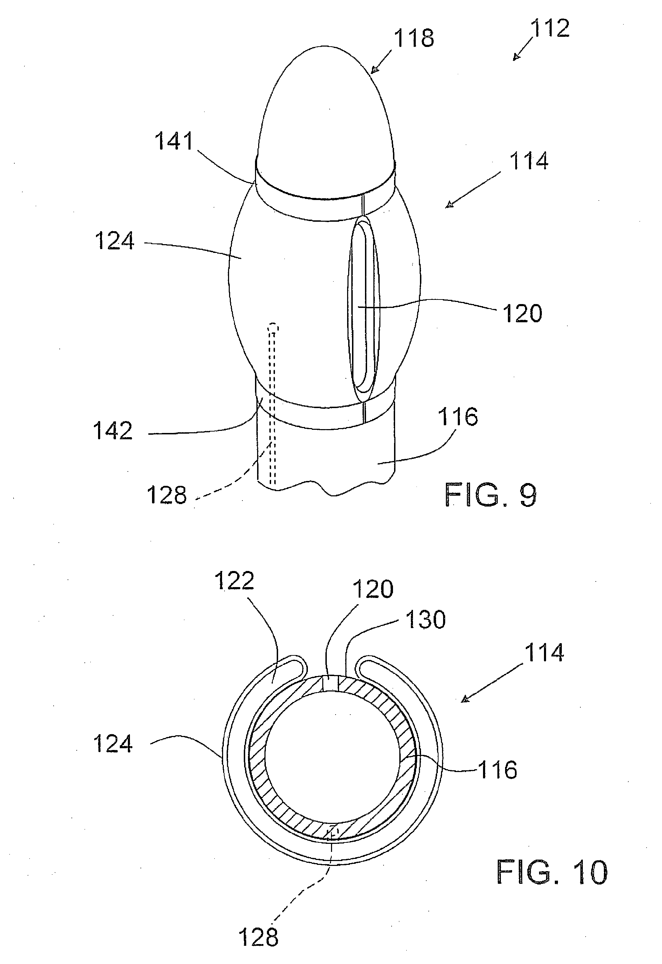

A first example of this is schematically shown in the embodiment according to Figures 9 and 10.

For the sake of simplification, parts in this embodiment which are similar to corresponding parts in the previous embodiment will be indicated by the same numbering as in the previous embodiment, but prefixed with "1".

With reference to Figure 9, the catheter 112 is therefore formed by a flexible tube 116 which terminates in a closed top end 118. The catheter has an axially elongated hole or slit 120 close to the top end 118.

The self-locating device 114 (shown partially inflated in Figure 9) is formed with an elastic membrane 124 which is suitably extended and which is closed in a loop so as to form a casing which defines and encloses the chamber 122. Advantageously, this may be obtained by using a short tubular section which is closed at the ends by means of welding or gluing so as to have a "bag-like" form. As can be clearly seen in Figures 9 and 10, the device 114 is advantageously formed with a width similar to the circumference of the catheter tube so as to be able to be wound over a circular rim portion around the flexible tube 116 at the height of the hole 120, leaving the zone 130 exposed, and is fixed to the wall of the flexible tube. The height of the device.is advantageously similar to the axial length of the elongated hole. As with the previous embodiments, the hole may also extend, at least at the bottom, beyond the length of the self-locating device. Advantageously, fixing is performed at least along the opposite top circumferential edge 141 and bottom circumferential edge 142 thereof (advantageously coinciding with the closing welds or bonds which, if desired or appropriate, may also be performed at the same time as fixing to the tube). The circumferential edges may be situated slightly above and below the slit 120 (as shown in the Figures) or, as can be easily imagined, may be arranged alongside its axial ends.

The interior of the chamber 122 is connected to the duct 128 for supplying the inflation fluid. This connection may be performed using varied means which can be easily imagined by a person skilled in the art in the light of the description of the invention provided herein. The duct 128 is in turn connected to the fluid supplying and discharge means as shown for the embodiment according to Figure 2.

As is clear from Figure 10, the device 114 may for example be sealingly fixed to the wall of the flexible tube also around the outlet of the duct 128 opposite which, in the wall of the membrane 124, a suitable passage for connecting the duct 128 and the chamber 122 is formed. A thin connecting tube (mounted or integral), not shown, may also be used, said tube, from the outlet of the duct 128, penetrating sealingly into the chamber 122 through the wall of the membrane 124.

As is clear from Figure 9, the self-locating device may also be constructed in the form of a sleeve 124 (instead of being wound around) and fitted via the end 118 of the catheter so as to be fixed along the edges 141 and 142, which are circumferentially continuous. Obviously, the sleeve may have a double wall, as shown in Figure 10, or a single wall, as shown in Figure 5. The opening in the circular segment which exposes the surface zone with the drainage hole may be formed in the sleeve during formation of the sleeve, for example as a cut in the tubular membrane wall which forms it. Closure of the chamber along the edge of this opening may be performed for example by means of welding or gluing of the edge to the wall of the catheter tube. The opening in the membrane or drainage hole in the catheter wall may also be formed simultaneously, for example by means of punching and welding along the edge.

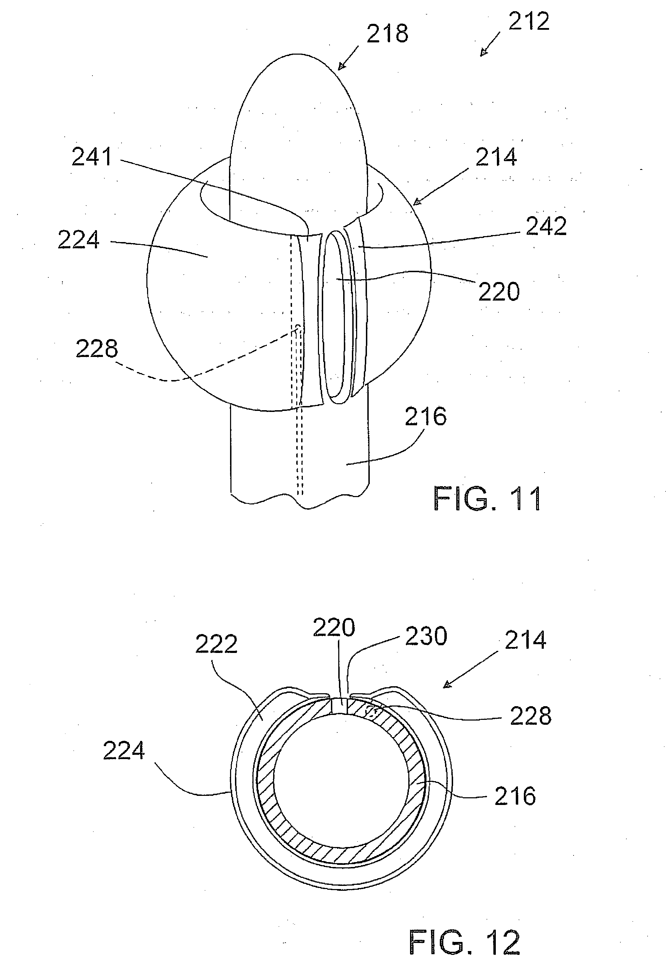

Figures 11 and 12 shows a further alternative embodiment of the catheter according to the invention.

For the sake of simplification, parts in this embodiment which are similar to corresponding parts in the previous embodiment will be indicated by the same numbering as in the previous embodiment, but prefixed with "2".

With reference to Figure 11, the catheter 212 is therefore formed by a flexible tube 216 which terminates in a closed top end 218. The catheter has an axially elongated hole or slit 220 close to the top end 218.

The self-locating device 214 is formed with an elastic membrane 224 which is suitably extended and which is closed in a loop so as to form a casing which defines and encloses the chamber 222. In a similar manner to the previous embodiment it is possible to use a suitable short tubular section which is closed at the ends by means of welding or gluing so as to have a "bag-like" form.

As can be clearly seen in Figures 11 and 12, the device 214 is advantageously formed with a length similar to the circumference of the catheter tube so as to be able to be wound over a circular rim portion around the flexible tube 216 at the height of the hole 220, leaving the zone 230 exposed, and is fixed to the wall of the flexible tube. The height of the device is advantageously similar to the axial length of the elongated hole. As with the previous embodiments, the hole may also extend; at least at the bottom; beyond the length of the self-locating device. Fixing of the device is performed at least along its two opposite edges 241 and 241 which extend in an axial direction with respect to the tube along the opposite sides of the hole 220. Advantageously, these fixing edges coincide with the closing welds or bonds of the ends of the tubular element forming the device 214 and, if desired, may also be performed at the same time as fixing of the element to the tube.

The interior of the chamber 222 is connected to the duct 228 for supplying the inflation fluid. This connection may be performed using varied means which can be easily imagined by a person skilled in the art in the light of the description of the invention provided herein. The duct 228 is in turn connected to the fluid supplying and discharge means as shown for the embodiment according to Figure 2.

As is clear from Figures 11 and 12, in order to provide the connection between chamber and duct, the device 214 may for example be sealingly fixed to the wall of the flexible tube around the outlet of the duct 228 opposite which, in the wall of the membrane 224, a suitable passage for connecting the duct 228 and the chamber 222 is formed.

Advantageously the fixing zone may extend alongside and parallel to one of the fixing edges 241 or 242, as clearly shown in broken lines in Figure 11. As a result it is possible to fix, for example, to the wall of the tube 216 a lip of the membrane, not yet closed, comprising also the passage towards the duct 228 so as to then close onto this lip the other lip of the membrane in order to form the closed edge 241.

As in the previous embodiments, it is also possible to use a thin connecting tube (mounted or integral), not shown, which from the outlet of the duct 228, penetrates sealingly into the chamber 222 through the wall of the membrane 224.

As can be seen in Figure 11 (where the self-locating device is shown in the inflated condition), as a result of the device 214 being fixed on the two sides of the slit 220, the slit 220 advantageously expands when the device is inflated by means of the fluid supplied into the chamber 222.

At this point it is clear how the objects of the invention are achieved, providing catheters which allow complete evacuation of the cavity into which they are introduced, while also guaranteeing an effective self-locating action.

Obviously, the above description of embodiments which apply the innovative principles of the present invention is provided by way of illustration of these innovative principles and must therefore not be regarded as limiting the scope of the rights claimed herein.

For example, the wall of the self-locating devices 114 or 214 which is directed towards the outer wall of the catheter tube may also be entirely fastened to this catheter wall, if preferred or required. The casing which forms the device may also be realized in the manner of a "bubble", suitably shaped, as well as a bag which is closed along its edges. Moreover, in all the embodiments it is possible to apply measures which will be obvious to the person skilled in the art for reducing or eliminating any step which could hinder insertion or extraction of the catheter. In particular, especially in the case of a "bag-like" or "bubble-like" configuration of the self-locating device, a seat which is suitably inset in the outer wall of the catheter may be provided. Using known production techniques it is also possible to envisage forming the device integrally with the flexible tube of the catheter. The duct supplying the inflation fluid may also be associated with the catheter tube in another manner, apart from being formed in the thickness of its wall.

The materials used may be chosen from among those normally used for similar applications, for example silicone, polyurethane, etc.

In one variant of the embodiments according to the invention, the membrane may also extend so as to cover a zone of the flexible tube extending further than the section where the self-locating device is formed. The extension of the membrane may also be such as to cover it along its entire length. This is schematically shown in broken lines in Figure 1.

In particular, the membrane 24 may have the same form described above in the zone defined by the self-locating device, while it will be welded on the surface of the flexible tube so as to create a duct 43 connecting the diametral chamber 22 to the inflation fluid supply means 26.

In this particular case the flexible tube does not require the duct 28 described previously.

Obviously, the exact form assumed by the self-locating device, once inflated, may be different from that schematically shown in the Figures, depending on various factors such as the inflation pressure, the elasticity and the membrane fixing and/or closing points, etc. For example, the cross-section of the chamber may be eccentric with respect to the axis of the catheter tube and also expand further on the opposite side to the drainage hole.

## Claims

1. Catheter structure equipped with an adjustable self-locating device, comprising a flexible tube (16,116,216) which has one end (18,118,218) intended to be introduced into the cavity to be evacuated and provided with a hole (20,120,220) which connects the interior of the cavity to be drained with the central drainage channel of the catheter, allowing evacuation thereof, a diametral chamber (22,122,222) which is formed by a membrane (24,124,224) expandable substantially towards the outside of the flexible tube (16,116,216) and which surrounds over a predetermined height the outer surface of said flexible tube (16,116,216), and a duct (28,43,128,228) which is associated with the flexible tube (16,116,216) and connects said diametral chamber (22,122,222) to means for supplying and controlled discharge of a pressurised fluid, said diametral chamber (22,122,222) extends over a circular rim portion around the flexible tube (16,116,216) which is interrupted in a single surface zone (30,130,230) of said flexible tube which is thus devoid of diametral chamber (22,122,222) and in which said hole (20,120,220) is formed, **characterized in that** the hole (20,120, 220) has its bottom edge situated partially underneath the bottom edge of said diametral chamber (22,122,222), and the membrane (24) consists of a single continuous element in the transverse plane in the form of a circumferential sleeve which is arranged on the tube for enclosing the chamber, the sleeve being welded to the flexible tube (16), along two circumferences in two suitably spaced transverse planes, and in the zone (30) without diametral chamber, and having an opening opposite the said surface zone in the welded zone which comprises the hole so as to allow communication between the hole and the exterior.

2. Catheter structure according to claim 1, wherein the membrane (24) covers the flexible tube along the whole of its axial length and said membrane is welded to the flexible tube except in the zone where it forms the adjustable self-locating device (14).

3. Catheter structure according to the preceding claim, wherein the membrane (24) has a non-welded zone which extends between the bottom surface (34) of the diametral chamber (22) and the means (26) for supplying and discharging a pressurised fluid, so that the fluid connection between the diametral chamber (22) and the means (26) for supplying and discharging pressurised fluid is allowed.

4. Catheter structure according to Claim 1, wherein the duct (28,128,228) is formed in the thickness of the flexible tube wall extending so as to emerge at a point inside the chamber.

5. Catheter structure according to Claim 1, wherein with respect to a transverse plane, the angle formed by a straight line tangential to the membrane and a straight line tangential to the tube, in the zone where the membrane is separated from the tube, is less than 90° when the membrane is expanded to form the chamber.

6. Catheter structure according to Claim 1, wherein when the membrane is expanded to form the chamber, the cross-section of the chamber is eccentric with respect to the axis of the catheter tube and also expanded further on the opposite side to the drainage hole.

## Patentansprüche

1. Kathederstruktur mit einer einstellbaren, selbst-anordnenden Einrichtung, welche aufweist, ein flexibles Rohr (16, 116, 216) mit einem Ende (18, 118, 218), das in einen zu entleerenden Raum eingebracht werden soll, und mit einem Loch (20, 120, 220), das das Innere des zu entleerenden Raums mit dem zentralen Ablaufkanal des Katheders verbinder, was eine Entleerung ermöglicht, eine diametrale Kammer (22, 122, 222), die aus einer Membran (24, 1240, 224) gebildet wird, die im Wesentlichen auf die Aussenseite des flexiblen Rohrs (16, 116, 216) ausdehnbar ist und die die äußere Oberfläche des flexiblen Rohrs (16, 116, 216) in einer bestimmten Höhe umgibt, und eine Leitung (28, 43, 128, 228), die milder flexiblen Rohr (16, 116, 216) assoziiert ist und die diametrale Kammer (22, 122, 222) mit Mitteln zum Zuführen und einer gesteuerten Entleerung eines unter Druck stehenden Fluids verbindet, worin sich die diametrale Kammer (22, 122, 222) über einen ringförmigen Randbereich um das flexible Rohr (16, 116, 216) erstreckt, das in einem einzigen Ober-flächenbereich (30, 130, 230) des flexiblen Rohrs unterbrochen ist, und daher keine diametrale Kammer (22, 122, 222) aufweist, und in der das Loch (20, 120, 220) ausgebildet ist, **dadurch gekennzeichnet, dass** die Bodenkante des Lochs (20, 120, 220) teilweise unter der Bodenkante der diametralen Kammer (22, 122, 222) angeordnet ist, und dass die Membran (24) aus einem einzigen kontinuierlichen Element in einer transversalen Ebene in Form einer Umfangsbuchse besteht, die auf dem Rohr angeordnet ist, um die Kammer zu umgeben, worin die Buchse an das flexible Rohr (16) entlang zwei Umfangsbereichen in zwei geeignet beanstandeten transversalen Ebenen und in dem Bereich (30) ohne diametrale Kammer angeschweißt ist, und eine Öffnung aufweist, die dem Oberflächenbereich in der Schweisszone, die das Loch umfasst, abgewandt ist, um so eine Verbindung zwischen dem Loch und dem Äußeren ermöglicht.

2. Kathederstruktur nach Anspruch 1, worin die Membran (24) das flexible Rohr entlang dessen Längsachse bedeckt und worin die Membran an das flexible Rohr angeschweißt ist, mit der Maßgabe in dem Bereich, an dem sie die einstellbare, selbstanordnende Einrichtung (14) ausbildet.

3. Kathederstruktur nach einem der vorstehenden Ansprüche, worin die Membran (24) keinen Schweißbereich aufweist, der sich zwischen der Bodenfläche (34) der diametralen Kammer (22) und den Mitteln (26) zum Zuführen und Entleeren eines unter Druck stehenden Fluids erstreckt, so dass die Fluidverbindung zwischen der diametralen Kammer (22) und den Mitteln (26) zum Zuführen und Entleeren des unter Druck stehenden Fluids ermöglicht wird.

4. Kathederstruktur nach Anspruch 1, worin die Leitung (28, 128, 228) in der Dicke der Wandung des flexiblen Rohrs ausgebildet ist, und sich erstreckt, um so an einem Punkt in der Kammer hervorzutreten.

5. Kathederstruktur nach Anspruch 1, worin hinsichtlich einer transversalen Ebene der durch eine gerade Linie tangential zu der Membran und einer geraden Linie tangential zu dem Rohr gebildete Winkel in dem Bereich, an dem die Membran von dem Rohr getrennt ist, kleiner als 90 ° ist, wenn die Membran ausgedehnt ist, um die Kammer auszubilden.

6. Kathederstruktur nach Anspruch 1, worin wenn die Membran unter Bildung der Kammer ausgedehnt ist, der Querschnitt der Kammer hinsichtlich der Achse des Kathederrohrs ekzentrisch ist und auch weiter auf die abgewandte Seite des Abflusslochs ausgedehnt ist.

## Revendications

1. Structure de cathéter équipée avec un dispositif d'auto-positionnement ajustable, comprenant un tube flexible (16, 116, 216) qui a une extrémité (18, 118, 218) prévue pour être introduite dans la cavité à évacuer et prévue avec un trou (20, 120, 220) qui raccorde l'intérieur de la cavité à drainer avec le canal de drainage central du cathéter, permettant son évacuation, une chambre diamétrale (22, 122, 222) qui est formée par une membrane (24, 124, 224) expansible sensiblement vers l'extérieur du tube flexible (16, 116, 216) et qui entoure, sur une hauteur prédéterminée, la surface externe dudit tube flexible (16, 116, 216), et un conduit (28, 43, 128, 228) qui est associé avec le tube flexible (16, 116, 216) et raccorde ladite chambre diamétrale (22, 122, 222) aux moyens pour l'alimentation et la décharge contrôlée d'un fluide sous pression, ladite chambre diamétrale (22, 122, 222) s'étend sur une partie de bord circulaire autour du tube flexible (16, 116, 216) qui est interrompu dans une zone de surface unique (30, 130, 230) dudit tube flexible qui est ainsi dépourvue de chambre diamétrale (22, 122, 222) et dans laquelle ledit trou (20, 120, 220) est formé, **caractérisée en ce que** le trou (20, 120, 220) a son bord inférieur situé partiellement au-dessous du bord inférieur de ladite chambre diamétrale (22, 122, 222), et la membrane (24) se compose d'un seul élément continu dans le plan transversal sous la forme d'un manchon circonférentiel qui est agencé sur le tube pour entourer la chambre, le manchon étant soudé au tube flexible (16), le long de deux circonférences dans deux plans transversaux sensiblement espacés, et dans la zone (30) sans chambre diamétrale, et ayant une ouverture opposée à ladite zone de surface dans la zone soudée qui comprend le trou afin de permettre la communication entre le trou et l'extérieur.

2. Structure de cathéter selon la revendication 1, dans laquelle la membrane (24) recouvre le tube flexible le long de la totalité de sa longueur axiale et ladite membrane est soudée au tube flexible, excepté dans la zone où il forme le dispositif d'auto-positionnement ajustable (14).

3. Structure de cathéter selon la revendication précédente, dans laquelle la membrane (24) a une zone non soudée qui s'étend entre la surface inférieure (34) de la chambre diamétrale (22) et les moyens (26) pour alimenter et décharger un fluide sous pression, de sorte que le raccordement de fluide entre la chambre diamétrale (22) et les moyens (26) pour alimenter et décharger le fluide sous pression est autorisé.

4. Structure de cathéter selon la revendication 1, dans laquelle le conduit (28, 128, 228) est formé dans l'épaisseur de la paroi de tube flexible s'étendant afin de ressortir au niveau d'un point à l'intérieur de la chambre.

5. Structure de cathéter selon la revendication 1, dans laquelle, par rapport à un plan transversal, l'angle formé par une ligne droite tangentielle à la membrane et une ligne droite tangentielle au tube, dans la zone où la membrane est séparée du tube, est inférieur à 90° lorsque la membrane est expansée afin de former la chambre.

6. Structure de cathéter selon la revendication 1, dans laquelle, lorsque la membrane est expansée afin de former la chambre, la section transversale de la chambre est excentrique par rapport à l'axe du tube de cathéter et également expansée davantage sur le côté opposé au trou de drainage.
